# EUROPEAN PATENT SPECIFICATION

(11) **EP 3 273 864 B1**
(45) Date of publication and mention of the grant of the patent: **14.06.2023**
(21) Application number: 16716315.3
(22) Date of filing: 23.03.2016
(51) Int. Cl.: A61B 10/00, A61B 5/20, G01F 9/00

(54) **URINE SPECIMEN COLLECTION SYSTEM**
HARNPROBENSAMMELSYSTEM
DISPOSITIF DE COLLECTION D'UN ECHANTILLION D'URINE

(30) Priority: 25.03.2015 TR 201503588; 17.04.2015 TR 201504747; 08.03.2016 TR 201603058; 15.03.2016 TR 201603360
(43) Date of publication of application: 31.01.2018
(73) Proprietor: Oruba Medikal Teknoloji Arge Ic ve Dis Tic. Ltd. Sti., Ankara (TR)
(72) Inventor: ULUSAHIN, Utku, Ankara (TR)
(74) Representative: Berkkam, Ayfer
(86) International application number: PCT/TR2016/050075
(87) International publication number: WO 2016/153452

(56) References cited:
- EP-A1- 1 593 345
- WO-A1-90/03149
- WO-A1-90/13797
- WO-A1-2013/128376
- US-A- 4 891 993
- US-A1- 2005 247 121

## Description

### Technical Field

The present invention relates to an urine specimen collection system in which an urine sample to be taken for laboratory tests, can be collected without interrupting the person while urinating.

### Prior Art

Urine can be considered as a sterile body secretion for a healthy individual. Chemical structure of the urine that is formed as a result of filtration of toxins and metabolic wastes in the body by the kidneys is altered because of the infections and physical degradations occurring in the urinary system. Because the urinary test results enable the physicians to gain insights about many issues, it is the second most desire test by the physicians.

In order to perform the test, urine sample is demanded from patients in a specimen container. Transporting the specimen to be examined in the laboratory, in to a urine specimen container will be performed by the patient. Therefore, the patient has to urine into a container which he also holds by hand. This unhygienic situation can also compromise the hygiene of the environment. At the same time, in order to obtain accurate test results, urine sample collection process should be hygienic. An example of urine sample collector is disclosed in United Kingdom patent application GB2162312A. In this application, according to the principle of computational fluid, an individual transport his/her urine into a container through a funnel. Then, filling of urine into a specimen container which has the same base height, is waited. Obtained specimen tube is removed by pulling out from the center of the base and the obtained specimen is transported to a test tube by transported to pull out with a syringe. The collection of urine sample into the container is performed by individuals.

At the same time, in certain conditions, some patients urination speeds who is required to submit urine samples, is also measured for the diagnosis and diagnosis can be made with the help of obtained results. An example of aforementioned processes known in the field is disclosed in patent application US2012226196. In this application, a single-use, inexpensive and disposable container is mentioned. With the help of this aforementioned container, the patients are directed during the urination and can cast away the container later.

Another example known in the field is disclosed in another United States patent application US2005256428. In this aforementioned application, the weight of the urine is measured gravimetrically during urination. With the help of this aforementioned measurement, the urination speed can be measured at the same time.

WO2013/128376A1 discloses a urine flow meter detecting the urine level in a container by a weight transducer or a capacitance sensor. A solenoid valve adapted to start and stop rinsing of the container and a siphon for flushing the container are also disclosed.

WO90/13797A1 discloses a urine collection monitor having a variable capacitor sensitive to the amount of urine, a tube for drawing a urine sample and a drain.

US2005/247121A1 discloses a urine collection and monitoring system having optical means for detecting the level of urine and a pump operated to empty the vessel when the level of urine is detected to reache an upper optical means. The pump is operated until the level of urine is detected to fall below a lower optical means.

US4891993A discloses an apparatus and method for determining the beginning and cessation of urine flow by introducing a stream of urine to pass by electrodes thus changing the resistivity.

### Brief Description of the Invention

The invention is defined in claim 1, while preferred embodiments are shown in claims 2-5.

The present invention relates to urine collection system in which the required urine specimen is collected during the urination speed measurement. The present invention is a system that can automatically transport the required amount of urine from the container in which the urine is collected for urination speed measurement to urine specimen container, during the urination process for urination speed measurement.

The system which will include a contemporary pissoir and/or water closet like urination reservoir collects the inbound urine from this reservoir into a measuring container. The datas obtained from the differences of the reservoir weight momentarily is used to calculate the urination speed. Then, during the cleaning process, required amount of urine located in the measuring container is transported in to the urine specimen container and thus, the required urine for the laboratory studies is obtained. Then, system performs cleaning stages and will be ready for the next patient.

Through the usage of both water and incrementally disinfectant materials in the aforementioned cleaning process, the present invention provides the hygiene of the system and increased cabin service life.

### Disclosure of the Invention

Urine Specimen Collection System established for fulfilling the purposes of the present invention is shown in the accompanying figures, in which;

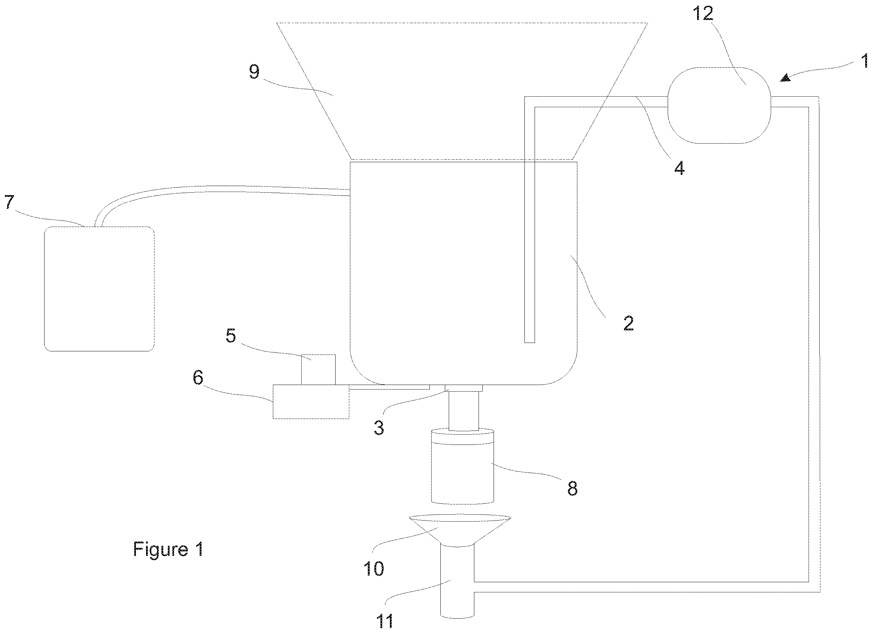

Figure 1 - Schematic illustration of the subject of the present invention, urine specimen collection system.

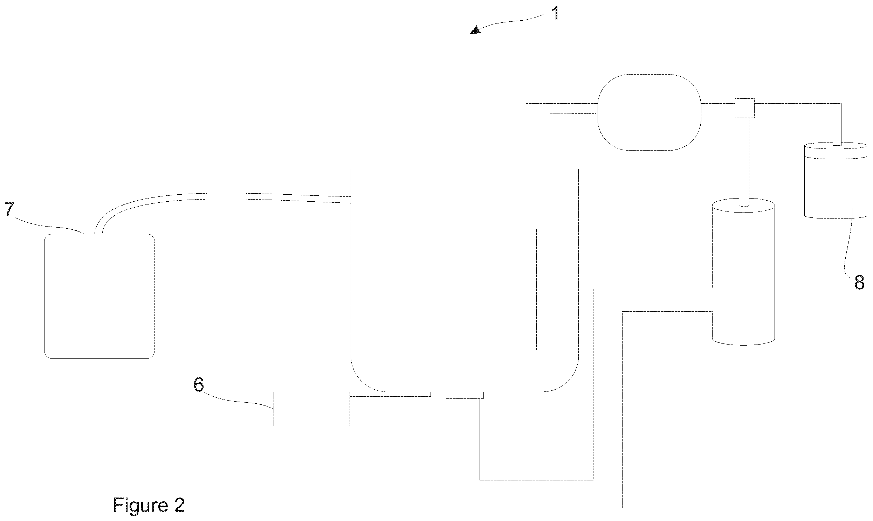

Figure 2 - Schematic illustration of Drain valve containing version of the subject of the present invention, urine specimen collection system.

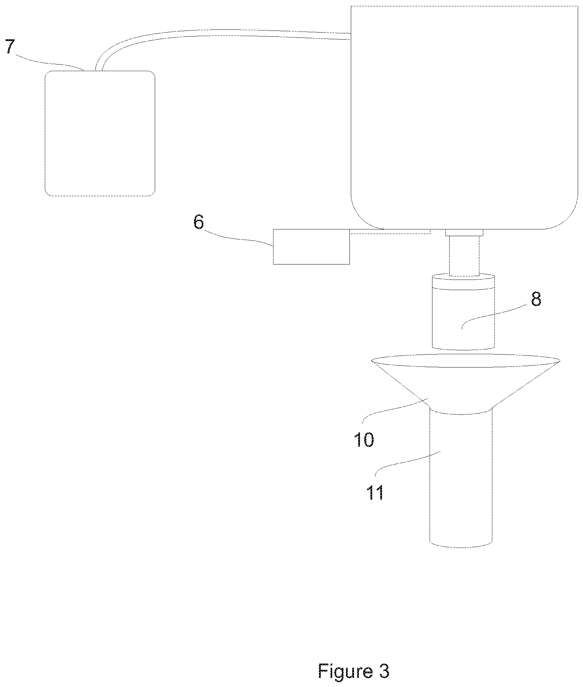

Figure 3 - Schematic illustration of single drain down version of the subject of the present invention, urine specimen collection system.

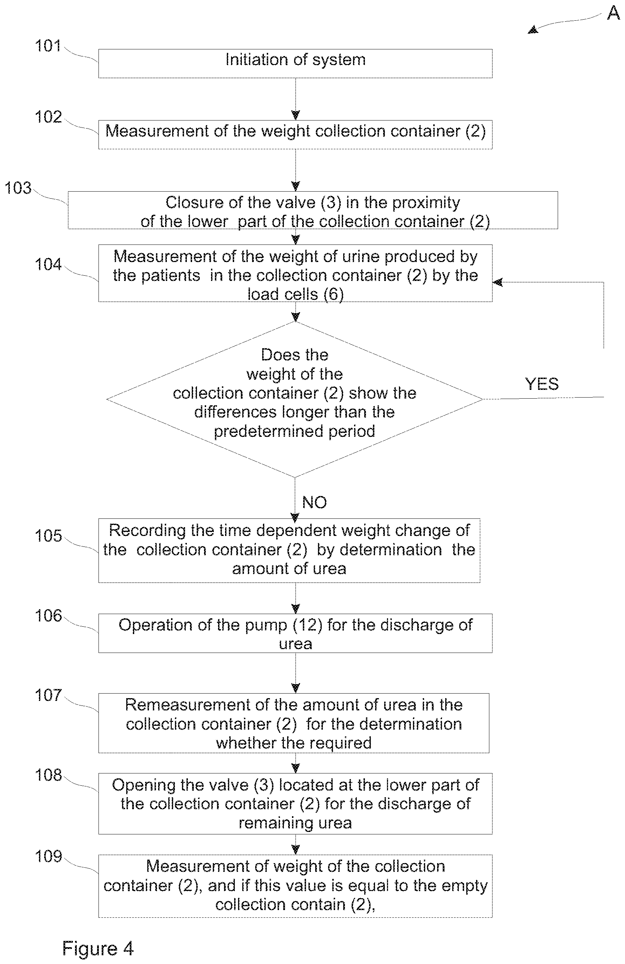

Figure 4 - Working algorithm of the subject of the present invention, urine specimen collection system.

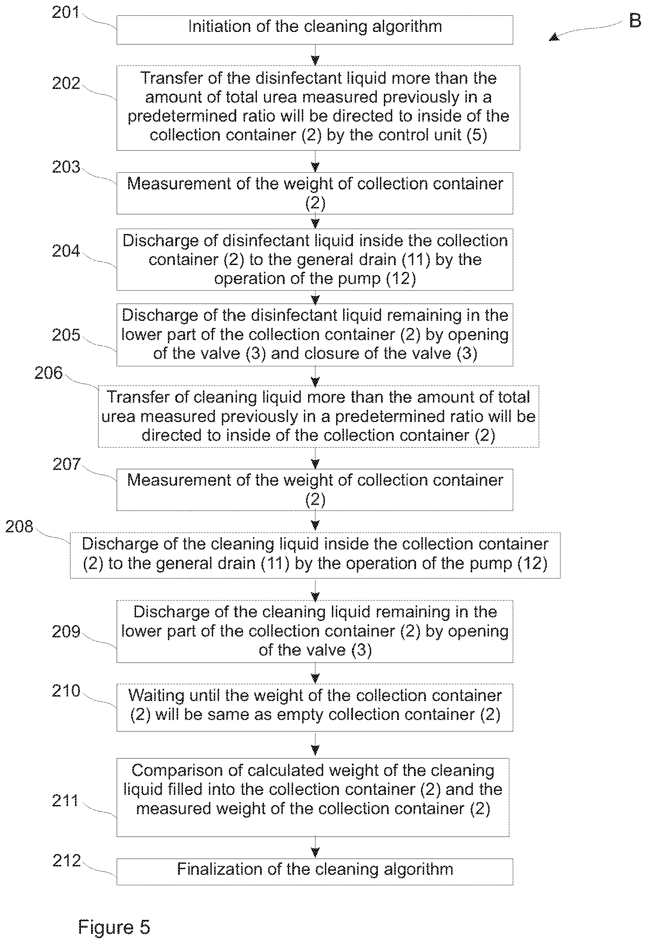

Figure 5 - Cleaning algorithm of the subject of the present invention, urine specimen collection system.

Parts in figures are numbered and the provisions of the numbers are given below.
1. Urine sample collection system
2. Collection container
3. Valve
4. Draining Pipe
5. Control unit
6. Load Cell
7. Tank
8. Specimen container
9. Reservoir
10. Funnel
11. General drain
12. Pump
A = Working Algorithm
B= Cleaning Algorithm

Urine specimen collection system (1), subject of the present invention, consists a collection container (2), a valve (2), a load cell (6), a pump (12), a specimen container (8) and a control unit (5) which records the weight information from the load cell (6) and determines the opening time of the valve in its principal form. While the patient to be analyzed urinates into the reservoir (9), urine produced by patient is directed into the collection container (2) and collected there. During this interval, weight of the collection container (2) is measured by load cell (6) momentarily, measurement values are recorded by control unit (5). Thus, with the finalization of urine flow of the patient to be analyzed, by the activation of the pump (12) by the control unit (5), the transfer of the urine collected into the collection container (2) to the general drain (11) by the drain pipe (4) is performed. After this disposal process, by the opening of the valve (3) located under the collection container (2), urine remaining into the collection container (2) is transferred into the specimen container (8). Then, cleaning processes is performed by the control unit (5). Thus, the urine sample of the patient who is tested for urination speed is also taken.

The time dependent graphic of weight increase recorded by the load cell (6) according to the urination speed of the patient, can be generated by the control unit (5). By the virtue of aforementioned information, gravimetrical measurement of flow rate is accomplished.

In order to compare the accuracy of the measurements of the load cell (6) that performed gravimetric measurement, a liquid with known density is employed. Liquid, stored in the aforementioned liquid tank (7) and used for cleaning of the remaining urine residues in the collection container (2) may be water, antiseptic or disinfectant liquid. Besides, it is also possible that rather than liquid is supplied from the tank (7), it can also be directly supplied from the municipal water system. It is possible that this liquid filled into a container with a constant velocity and measuring of the filling speed and total weight by the load cell (6) and determination of calibration values considering the consistence of these values with the previously set values.

In order to present invention operate the aforementioned processes, there is control unit (5) present on the invention. Aforementioned control unit (5) can measure the weight of the collection container (2) with the load cell (6) and can switch the valve (3). Control Unit also can control the pump (12) working for cleaning processes. Control unit (5) also performs the aforementioned calibration. Control unit (5) operates these components whose mode of operation is disclosed previously here, according to a working algorithm (A).

Collection container (2) is basically a container which the urine of the patients who use urine specimen collection system (1) collected into. There will be a valve (3) controlled by the control unit, beneath the collection container (2). In different embodiments of the invention, this valve (3) is used for the transfer of the urine in the collection container (2) to specimen container (8) or general drain (11). Additionally, during the cleaning process, the cleaning liquid will be found in the collection container (2) will be discharged with this valve (3). Weight of collection container (2) will be measured by the load cell located beneath of the collection container (2) momentarily. Obtained information will be recorded by transferring to control unit (5). Thus, the momentarily measurement of the patients urine flow is provided.

There will be a draining pipe (4) in a slouched configuration in the collection container (2). Through this draining pipe (4), the patients urine in the collection container (2) is disposed after the urination speed measurement processes. Besides, in different embodiments of the present invention, the draining pipe (4) will be used for disposal of the cleaning liquid. Urine or the cleaning liquid inside the collection container (2) will be directed to general drain (11) or specimen container (8) with the help of a pipe (12). In the pumped disposal version of the present invention, since the transfer of the urine specimen in the collection container (2) to the specimen container (8) is performed through the valve (3) located beneath of the collection container (2), the draining pipe (4) will located higher than the base of the collection container (2). With this height, volume of the cylinder formed the base surface of the collection container (2) will be smaller than volume of the specimen container (8). Thus, when the draining pipe (4) discharge the urine located in the collection container (2) with the help of the pump (12), it is provided that the amount of urine required to fill the specimen container (8) will be left in the collection container (2).

There will be (1) a tank (7) filled with disinfectant liquid in the urine specimen collection system for the functioning of cleaning algorithm (B). This tank (7) can be connected to municipal water supply. Thus, when the necessary measurement processes are completed, system (1) can use the liquid inside the tank (7) for self-cleaning. Additionally, since the amount water used for cleaning in the tank (7) is known, this information can be used in the calibration of urine specimen collection system.

When the urine collection system (1) working algorithm (A) is started (101), system will measure the weight of the collection container (2) (102). Weight of the collection container (2) can be different than the prespecified value because of solid or liquid residues remaining on the collection container (2). Thus, the weight of the collection container should be measured before every use. it is provided that this measurement value will be the reference weigh value in the control unit (5). Closure of the valve (3) located in the proximity of the base of the collection container (2) will be provided for urine flow rate measurements can be performed on the urine collected in to the collection container (2) (103). The audio and/or visual warning for noticing the patients that the system is ready for operation can be applied at this stage.

The amount of urine collected by the direction of patients urine in to the collection container (2) will be measured by the load cell (6) (104). Adjustment of the frequency of weight information gathered by the load cell (6) and the time dependent weight change of the collection container (2) will be governed by the control unit (5). Time dependent weight measurements will be performed on the collection container (2) until there won't be any change in weight of the predetermined time frame. If more time passes than the predetermined period without a change in weight, it is assumed the patients urination is ceased. Then, it is possible than the user can be notified by audio and /or illuminated way about the measurement process is over or there won't be any new measurements taken.

In order to calculate the collected amount of urine accumulated into the collection container (2), the difference between first and last weight of the container (2) and the time dependent weight increase of the container (2) will be recorded (105). Thus, time dependent change in the urination speed of the patient is determined and required information for urination speed analysis is recorded. After the recording processes, pump will be activated for the discharge of the urine first (106). Thus, urine located in the container will be discharged to general drain (11) with the help of the pump (12) through the draining pipe (4). Since the draining pipe (4) is in a slouched configuration standing higher than the base of the collection container (2), it is expected that there will be urine in this volume left in the collection container (2). By measuring the collection container (2) again by the load cell (6), it is determined that whether the remaining volume of urine left in the container (2) is suitable for the specimen container (8) (107). Then, by opening of the valve (3) located beneath the collection container (2), remaining urine in the low end of the collection container (2) will be transferred to specimen container (8) (108). After, waiting for a predetermined period for transfer of urine into the specimen container (8), the weight of the collection container (2) will be measured again and it is ensured that little or any urine left inside and valve(3) will be closed prior to the cleaning process (108).

Hereinabove, working algorithm (A) of the urine specimen collection system's (1) during the measurements taken from the patient is described in detail. Nonetheless, after every time it is used it is required both for hygiene and accuracy of measurements that the urine specimen collection system (1) is become ready. For this purpose cleaning algorithm (B) will be operated (201).

Cleaning algorithm (B) is required for the discharge of residual urine and sterilization of the collection container (2) for the next specimen collection process. After the cleaning algorithm (B) is started by control unit (5) (201), disinfectant liquid stored into the tank (7) will be directed to the collection container (2) (202). The amount of disinfectant liquid will be determined according to the total amount of urine measured during the operation of working algorithm (A) (105). Because of the possibility of splashing of urine to the higher part of the collection container(2), disinfectant liquid more than the amount of total urine measured in a predetermined ratio will be directed to inside of the collection container (2) from the tank (7) (202). In order to clean the bacteria, viruses and fungi residues which possibly found into the urine, alcohol or derivatives can be used as disinfectant liquid.

After the transfer of the disinfectant liquid into the collection container (2) process, (202), the weight of the collection container (2) will be measured (203). Thus, it will be determined the volume of disinfectant liquid taken into the collection container (2) since the weight density of the disinfectant liquid is known.

Then, for the discharge of the disinfectant, pump (12) will be operated first (204). Thus, sterilization of the draining pipe (4) will be also performed with the help of the pump (12). Disinfectant located into the collection container (2) will be discharged into the general drain (11) with the help of the pump (12). Then, the valve (3) located beneath of the collection container (2) , remaining urine in the lower part of the collection container (2) will be discharged into general drain (11) and valve (3) will be closed after waiting for predetermined period (206).

Then, in order to clean the urine specimen collection system (1) and purification of it from the disinfectant liquid, cleaning liquid will be directed into collection container (206). Required amount of cleaning liquid will be determined according to total amount of urine measured during the operation of working algorithm (A) (105). Because of the possibility of splashing of urine to the higher part of the collection container (2), cleaning liquid more than the amount of total urine measured in a predetermined ratio will be directed to inside of the collection container (2) (206). After these step, the weight of the collection container (2) will be measured (207).

Then, for the discharge of the cleaning liquid, pump (12) will be operated first (208). Thus, cleaning of the draining pipe (4) in which urine and disinfectant liquid passes inside, will be also performed with the help of the pump (12). Cleaning liquid located into the collection container (2) will be discharged into the general drain (11) with the help of the pump (12) (208). Then, the valve (3) located beneath of the collection container (2) , remaining urine in the lower part of the collection container (2) will be discharged into general drain (11) (209). After this step, it will be allowed that the weight of the collection container (29 will be equal to the weight of the empty collection container (2) (210). Thus, it is ensured that the collection container (2) is totally empty. After the complete discharge of the container(2), valve (3) will be closed after waiting for predetermined period (206).

The weight of the collection container (2) filled with the cleaning liquid will be measured in order to perform the urine specimen collection system's (1) own calibration (207). The weight of collection container (2) filled with cleaning liquid measured in weight measurement step (207) and the known weight of the cleaning liquid filling into the collection container (2) will be compared by control unit (5) (211). If the difference is bigger than the predetermined value, urine specimen collection system (1) is determined that the difference is due to the miscalculation performed by the load cell (6), give a warning and shut it down. if the difference measured is lower than the predetermined value, the system assumed that the difference is due to a momentarily change and the value will be used for calibration. Calibration of the urine specimen collection system (1) will be performed by control unit (5) and will be finalized by cleaning algorithm (212).

In the pumped version of the urine specimen collection system (1), urine, disinfectant liquid or cleaning liquid filled into the collection container (2) for cleaning and measurement is withdrawn by draining pipe (4) with the help of the pump (12). After this discharge, urine, disinfectant liquid or cleaning liquid is transported into the general drain (11). Urine remaining in the lower part of the collection container (2), is transported into specimen container (8) through the valve (3) located beneath the collection container. After the transfer of the urine, removal of specimen container (8) from inside of the urine specimen collection system (1) and beginning the operation of cleaning algorithm (B), the remaining disinfectant liquid or cleaning liquid into the collection container (2) is transported to general drain (11) through the opening of the valve (3) located beneath the collection container (2).

In the valved drain version of the urine specimen collection system (1), urine, disinfectant liquid or cleaning liquid filled into the collection container (2) for cleaning and measurement is withdrawn by draining pipe (4) with the help of the pump (12). During this discharge, the urine withdrawn from the collection container (2) first fills the specimen container (8) for a determined period and transported to general drain (11) by the pump (12) by the operation of a time dependent valve (13). Urine, disinfectant liquid and cleaning liquid remaining into the collection container (2) is directed to general drain (11) through the valve (3) located beneath the collection container (2).

In the single drain version of the urine specimen collection system (1), the urine collected from the patient for analysis collected into the collection container (2), is filled the specimen container(8) thorough the opening of the valve (3) located beneath the collection container (2) for a predetermined period. Then, after the removal of the specimen container(8) from the urine specimen collection system (1), by the opening of the valve(3) located beneath the collection container (2) by the control unit (5), remaining urine left in the collection container (2), can be discharged to the general drain (11). Thus, after the filling of the disinfectant liquid or cleaning liquid into the collection container (2), they are transferred to general drain (11) by the opening of the valve (3). In order to perform liquid transfers (cleaning and/or urine) to general drain when the valve (3) is open, funnel can be placed on the general drain (11).

Present invention can be used for the determination of the patients urination speed without collecting the specimens by replacing the step of opening of the valve (3) for the transfer of urine into the specimen container (8) (108) with opening of the valve (3) for the transfer of general drain (11) in the working algorithm (A) of the present invention. Then, there will be general drain (11) in place of specimen container (8) beneath the valve (3) located under the collection container (2).

Hereinabove, it is mentioned that the urine specimen collection system (1) can made audio and/or optic warnings to warn the patients. In order to monitor aforementioned warnings on the system, a touchscreen can be used. Aforementioned screen can possibly include the display of the graphics entire measurement period and information and warnings explaining operation of the system. A printer in the vicinity of the system or on the system can be used for reporting the results of the measurements to the patient.

Besides the information can be recorded as anonymous if the patient demands the related report as an anonymous user, if the patient demands the report by signing in his/her name, the related information can be made reachable to the patient and his/her physician.

In addition to aforementioned, diabetes can also be diagnosed over urine. Besides the urination speed measurements and urine density measurements, it is also possible to measure glucose values of the patients by employing the urine specimen collection system (1). Required urine specimen for these aforementioned measurements can be taken from inside the collection container (2) or draining pipe (4) by employing a receiver.

it is mentioned here that the present invention can be touch-operated for performing the aforementioned interactions. It is possible that patients may have concerns about touching the surface because the place of use of the present invention. Thus, it can be possible that by employing a proximity sensor (proximity switch) rather than touch-screen or button, the patient can control the properties of the system such as initiation of the system, shut down the system, request the printed document, without touching the system. For alleviation of the high costs of loss of the printed documents and printer and screen, it can be possible that all the information can be transferred to patients mobile devices and patients can use these mobile devices. Mapping of the software to be loaded into the mobile device and urine specimen collection system (1) can be done over OR code, NFC, Bluetooth, WiFi. After the mapping, patient can govern all the functions of the urine specimen collection system (1) and download all the outputs generated by this process into his/her mobile device.

The urine specimen collection system (1) can be initiated via barcodes already in use in hospitals for attaching onto the test specimens collected from patients. A barcode scanner can be placed onto the urine specimen collection system (1) for this purpose. After this aforementioned initiation, by recording the test result onto the barcode, it will become possible that all the information can be recorded into the hospital automation system with this number.

## Claims

1. An urine specimen collection system (1) having a control unit (5) which is able to execute a working algorithm (A) for the usage during the urination of a person, the working algorithm (A) consisting of the following steps;
- Measurement of the weight of a collection container (2) (102),
- Closure of a valve (3) in the proximity of the lower part of the collection container (2) in order to perform measurements on the collected urine (103),
- Measurement of the weight of urine produced by patients in the collection container (2) by a load cell (6) (104),
- Recording of the difference between first and final weights and the time dependent weight change of the collection container (2) for the purpose of determination the amount of urine present in the collection container (105),
- Operation of a pump (12) for the discharge of urine (106),
- Opening of the valve (3) located at the lower part of the collection container (2) to discharge the remaining urine left in the collection container (2) and to transfer the remaining urine into a specimen container (8) for the collection of a urine specimen from the urine transferred from the patient into the collection container (2).

2. An urine specimen collection system (1) according to any of the preceding claims wherein the system further comprises a proximity sensor for interaction with the patient without touching.

3. An urine specimen collection system (1) according to any of the preceding claims, the system further comprising a cleaning algorithm (B) consisting of the following steps for the sterilization and cleaning of the urine specimen collection system (1) for the use of a second patient after the execution of working algorithm (A);
- Transfer of a disinfectant liquid more than the amount of total urine measured previously in a predetermined ratio, wherein the disinfectant liquid will be directed to the inside of the collection container (2) from a tank (7) (202),
- Discharge of the disinfectant liquid inside the collection container (2) to a general drain (11) by the operation of the pump (12) (204),
- Discharge of the disinfectant liquid remaining in the lower part of the collection container (2) by opening of the valve (3) and closure of the valve (3) (205),
- Transfer of a cleaning liquid more than the amount of total urine measured previously in a predetermined ratio, wherein the cleaning liquid will be directed to the inside of the collection container (2) (206),
- Discharge of the cleaning liquid inside the collection container (2) to the general drain (11) by the operation of the pump (12) (208),
- Discharge of the cleaning liquid remaining in the lower part of the collection container (2) by opening of the valve (3) (209),
- Waiting until the weight of the collection container (2) will be same as that of the empty collection container (2) (210).

4. An urine specimen collection system (1) according to claim 3 wherein, sterilization is performed by using municipal water supply rather than the disinfectant liquid supplied from the tank (7).

5. An urine specimen collection system (1) according to claim 3 wherein the cleaning algorithm (B) comprises further a step in which the weight of the collection container (2) filled with cleaning liquid (207) and the empty weight of the cleaning container (2) are compared, for the control of the proper functioning of the load cell (6) (211).

## Patentansprüche

1. Ein Urinprobenentnahmesystem (1) mit einer Steuereinheit (5), die in der Lage ist, einen Arbeitsalgorithmus während des Harnlassens einer Person auszuführen. Der Arbeitsalgorithmus (A) besteht aus den folgenden Schritten;
- Messung des Gewichts des Sammelbehälters (2) (102),
- Schließung des Ventils (3) in der Nähe des unteren Teils des Sammelbehälters (2), um Messungen an dem gesammelten Harn durchzuführen (103),
- Messung des Gewichts des von dem Patienten im Sammelbehälter (2) produzierten Urins durch die Lastzelle (6) (104),
- Aufzeichnung des Unterschieds zwischen dem ersten und dem letzten Gewicht sowie der zeitabhängigen Gewichtsänderung des Sammelbehälters (2) zur Bestimmung der im Sammelbehälter vorhandenen Harnstoffmenge (105),
- Betrieb der Pumpe (12) zur Entleerung des Harns (106),
- Öffnen des Ventils (3), das sich am unteren Teil des Sammelbehälters (2) befindet, um den verbleibenden Urin im Sammelbehälters (2) abzulassen und den verbleibenden Urin in einen Probenbehälter (8) zu übertragen, um eine Urinprobe von dem von dem Patienten in den Sammelbehälters (2) übertragenen Urin zu sammeln.

2. Ein Urinprobensammlungssystem (1) gemäß einem der vorhergehenden Ansprüche, welches einen Näherungssensor umfasst, der zur Interaktion mit dem Patienten ohne Berührung dient.

3. Ein Urinprobensammlungssystem (1) gemäß einem der vorherigen Ansprüche, **gekennzeichnet durch** ein Arbeitsalgorithmus (A), die folgenden Schritte für die Sterilisation und Reinigung des Urinprobensammlungssystem (1) für die Verwendung durch einen zweiten Patienten nach der Durchführung des Arbeitsalgorithmus (A) umfasst:
- Übertragung von Desinfektionsflüssigkeit in einem vorbestimmten Verhältnis, das mehr ist als die zuvor gemessene Gesamtmenge an Harnstoff, in den Sammelbehälter (2) aus dem Tank (7) (202),
- Entleerung der Desinfektionsflüssigkeit im Inneren des Sammelbehälters (2) in den allgemeinen Abfluss (11) durch Betätigung der Pumpe (12) (204),
- Entleerung der im unteren Teil des Sammelbehälters (2) verbleibenden Desinfektionsflüssigkeit durch Öffnen des Ventils (3) und Schließen des Ventils (3) (205),
- Übertragung von Reinigungsflüssigkeit in einem vorbestimmten Verhältnis, das mehr ist als die zuvor gemessene Gesamtmenge an Harnstoff, in den Sammelbehälter (2) (206),
- Entleerung der Reinigungsflüssigkeit im Inneren des Sammelbehälters (2) in den allgemeinen Abfluss (11) durch Betätigung der Pumpe (12) (208),
- Entleerung der im unteren Teil des Sammelbehälters (2) verbleibenden Reinigungsflüssigkeit durch Öffnen des Ventils (3) (209),
- Warten, bis das Gewicht des Sammelbehälters (2) dem Gewicht des leeren Sammelbehälters (2) entspricht (210).

4. Ein Urinprobensammlungssystem (1) gemäß Anspruch 3, **dadurch gekennzeichnet, dass** die Sterilisation durch Verwendung der Wasserversorgung der Gemeinde anstelle der Desinfektionsflüssigkeit aus dem Tank (7) durchgeführt wird.

5. Ein Urinprobensammlungssystem (1) gemäß Anspruch 3, **gekennzeichnet durch** einen Reinigungsalgorithmus (B), der aus einem Schritt besteht, bei dem das Gewicht des mit Reinigungsflüssigkeit gefüllten Sammelbehälters (2) (207) mit dem Gewicht des leeren Sammelbehälters (2) verglichen wird (211), um die ordnungsgemäße Funktion der Lastzelle (6) zu kontrollieren.

## Revendications

1. Un système de collecte d'échantillons d'urine (1) comprenant une unité de contrôle (5) capable d'exécuter un algorithme de fonctionnement (A) pendant la miction d'une personne, l'algorithme de fonctionnement (A) comprenant les étapes suivantes;
- Mesure du poids du récipient de collecte (2) (102),
- Fermeture de la valve (3) à proximité de la partie inférieure du récipient de collecte (2) afin d'effectuer des mesures sur l'urée collectée (103),
- Mesure du poids de l'urine produite par les patients dans le récipient de collecte (2) par la cellule de charge (6) (104),
- Enregistrement de la différence entre les poids initiaux et finaux et de la variation du poids en fonction du temps du récipient de collecte (2) dans le but de déterminer la quantité d'urée présente dans le récipient de collecte (105),
- Fonctionnement de la pompe (12) pour l'évacuation de l'urée (106),
- Ouverture de la valve (3) située à la partie inférieure du récipient de collecte (2) pour évacuer l'urine restante dans le récipient de collecte (2) et transférer l'urine restante dans un récipient d'échantillon (8) pour la collecte d'un échantillon d'urine à partir de l'urine transférée du patient au récipient de collecte (2).

2. Un système de collecte d'échantillons d'urine (1) selon l'une quelconque des revendications précédentes **caractérisé en ce qu'**il comprend un capteur de proximité pour interagir avec le patient sans contact.

3. Un système de collecte d'échantillons d'urine (1) selon l'une quelconque des revendications précédentes **caractérisé en ce qu'**il comprend un algorithme de nettoyage (A) comprenant les étapes suivantes pour la stérilisation et le nettoyage du système de collecte d'échantillons d'urine (1) pour l'utilisation par un deuxième patient après l'exécution de l'algorithme de travail (A);
- Transfert de liquide désinfectant supérieur à la quantité totale d'urée mesurée précédemment dans un rapport prédéterminé à l'intérieur du récipient de collecte (2) depuis le tank (7) (202),
- Évacuation du liquide désinfectant à l'intérieur du récipient de collecte (2) vers le drain général (11) par le fonctionnement de la pompe (12) (204),
- Évacuation du liquide désinfectant restant dans la partie inférieure du récipient de collecte (2) en ouvrant la soupape (3) et en fermant la soupape (3) (205),
- Transfert de liquide de nettoyage supérieur à la quantité totale d'urée mesurée précédemment dans un rapport prédéterminé à l'intérieur du récipient de collecte (2) (206),
- Évacuation du liquide de nettoyage à l'intérieur du récipient de collecte (2) vers le drain général (11) par le fonctionnement de la pompe (12) (208),
- Évacuation du liquide de nettoyage restant dans la partie inférieure du récipient de collecte (2) en ouvrant la soupape (3) (209),
- Attente jusqu'à ce que le poids du récipient de collecte (2) soit identique à celui du récipient de collecte vide (2) (210).

4. Un système de collecte d'échantillons d'urine (1) selon la revendication 3 **caractérisé en ce que** la stérilisation est réalisée en utilisant l'eau du réseau municipal plutôt que le liquide désinfectant provenant du tank (7).

5. Un système de collecte d'échantillons d'urine (1) selon la revendication 3, **caractérisé en ce qu'**il comprend un algorithme de nettoyage (B) consistant en une étape dans laquelle le poids du récipient de collecte (2) rempli de liquide de nettoyage (207) et le poids à vide du récipient de nettoyage (2) (211) sont comparés, pour le contrôle du bon fonctionnement de la cellule de charge (6).
